# EUROPEAN PATENT SPECIFICATION

(11) **EP 3 944 843 B1**
(45) Date of publication and mention of the grant of the patent: **26.07.2023**
(21) Application number: 20461557.9
(22) Date of filing: 30.07.2020
(51) Int. Cl.: A61F 13/14, A61B 17/135

(54) **PNEUMATIC COMPRESSION BANDAGE**
PNEUMATISCHE KOMPRESSIONS BANDAGE
BANDAGE DE COMPRESSION PNEUMATIQUE

(43) Date of publication of application: 02.02.2022
(73) Proprietor: MediNice Spolka Akcyjna, 02-103 Warszawa (PL)
(72) Inventor: STEC, Sebastian, 37-700 Przemysl (PL); CHOUDHARY, Sanjeev, 02-494 Warszawa (PL); KWIETNIAK, Karol, 07-440 Goworowo (PL)
(74) Representative: AOMB Polska Sp. z.o.o.

(56) References cited:
- EP-B1- 2 708 215
- WO-A1-2015/188154
- WO-A2-93/12708
- CN-A- 111 281 471
- US-A1- 2014 228 732
- US-A1- 2016 095 755

## Description

The subject of this invention is a pneumatic compression bandage intended in particular to be used in patients who underwent the implantation of medical devices used for cardiac electrotherapy to minimize or eliminate any hemorrhagic complications, primarily hematomas in the implantation site (device site). The bandage is used to prevent hematomas in the implantation sites of cardiac stimulation devices.

### Prior art

In the prior art, there is a device intended for applying compression to the sites to prevent hematomas after the procedure of medical device implantation in the site and the way of using the said device known under the application no. US 2008319473 (A1). Such a device may include cushions and relevant fasteners, e.g. in the form of a clamp, hinge, brace, clip, spring, a pneumatic or inflatable solution, a tape etc. However, the said device does not have an automatic control and pumping system to preset pressure values, responsible for maintaining the constant pressure.

The document CN111281471A discloses an electric air pressure hemostat which comprises a belt body; a first elastic band which is attached to one end of the band body; a second elastic band which is attached to the other end of the band body; a self-tightening hasp which is arranged between the first elastic band and the second elastic band and can enclose the belt body into a ring shape; a plurality of air bags which are arranged on the inner side of the belt body; an inflating device which is respectively communicated with the air bags through a plurality of breather pipes and can inflate the air bags; inflation flow valves which are arranged on the breather pipes and can control the inflation speed of the breather pipes. According to the electric air pressure hemostat, the belt body is fixed through the self-tightening hasp, the elastic bands can be wound through a winding shaft, the size of the belt body fixing ring can be changed at will, the electric air pressure hemostat is suitable for any part of the body, pressure is exerted on the hemostat through the air bags, and the pressure is adjustable, and the invention further provides a control method of the electric air pressure hemostat.

WO2015188154A1 is a document in which improvements in fluid volume measurement systems are disclosed for a pneumatically actuated diaphragm pump in general, and a peritoneal dialysis cycler using a pump cassette in particular. Pump fluid volume measurements are based on pressure measurements in a pump control chamber and a reference chamber in a two-chamber model, with different sections of the apparatus being modeled using a combination of adiabatic, isothermal and polytropic processes. Real time or instantaneous fluid flow measurements in a pump chamber of a diaphragm pump are also disclosed, in this case using a one-chamber ideal gas model and using a high speed processor to obtain and process pump control chamber pressures during fluid flow into or out of the pump chamber. Improved heater control circuitry is also disclosed, to provide added or redundant safety measures, or to reduce current leakage from a heater element during pulse width modulation control of the heater. Improvements are also disclosed in the application of negative pressure during a drain phase in peritoneal dialysis therapy, and to control the amount of intraperitoneal fluid accumulation during a therapy. Improvements in efficiency are also disclosed in the movement of fluid into and out of a two-pump cassette and heater bag of a peritoneal dialysis cycler, and in the synchronization of the operation of two or more pumps in a peritoneal dialysis cycler or other fluid handling devices using a multi-pump arrangement.

The document US5031604 discloses a device for applying compression to the patient's limb via a device in the form of a multi-chamber sleeve filled with pressurized fluid. The fluid pressure is increased to the required value by a compressor controlled via a control system which receives data from the manometer situated in the chamber and outside it. The device may fill the chamber to the preset pressure and increase or decrease it in the preset sequence. The compressor control algorithm used is the P-type algorithm based on comparing the pressure in the chamber to the atmospheric pressure. The disclosed control system switches the compressor on when the pressure in the chamber is lower than the preset one based on the ambient pressure or switches it off in the opposite situation.

The document no. WO2018213615A3 discloses a device similar to the one disclosed in the document no. US5031604 which has also sensors detecting the shot or fall, e.g. accelerometric ones. When the control system detects a possible hemorrhage caused by a fall or shot, it activates measures to increase the pressure in the device chamber to the preset value.

The document no. US5779657 discloses a device similar to the one disclosed in the document no. US5031604 which is also able to detect the hemorrhage onset and then to activate the device automatically and notify the healthcare practitioners thereof.

In the prior art, there is also a publication no. EP2708215B1, which discloses a pressure dressing, especially for preventing hemorrhagic complications after implanting medical devices in cardiac electrotherapy. Said dressing comprises attaching means and a pneumatic pressure cushion. It is characterized in that it comprises a pressure sensor connected with an electric control and pumping system provided with an integrated circuit for controlling pressure in the pressure cushion. EP2708215B1 is considered the closest prior art to the present invention. However, the said device does not have an automatic control and pumping system to preset pressure values, responsible for maintaining the constant pressure using a PID controller.

### The Essence of the Invention

The aim of this invention was to develop a significantly improved pneumatic compression bandage to minimize or eliminate any hemorrhagic complications, primarily hematomas in the implantation site (device site).

According to the invention, the pneumatic compression bandage particularly for preventing hemorrhagic complications following medical device implantations in cardiac electrotherapy with a cuff having a chamber to be filled with pressurized fluid. It has a harness to fasten the cuff in the wound area and a control and pumping unit connected pneumatically to the chamber in the cuff via a connection hose. The said control and pumping unit includes an electrical pump, an electromechanical valve connected electrically with the control and pumping unit, a pressure sensor connected electrically with the control and pumping unit. The said unit has a memory and a system capable of processing digital information, as well as a user interface in the form of buttons, light and sound indicators. The pneumatic compression bandage according to the invention is characterized by the fact that the controller of the pressure in the cuff chamber is of the PID type, i.e. it contains a proportional, integral and derivative part and is intended for controlling and configured to control the electrical pump to increase pressure in the cuff chamber and the valve to reduce pressure in the said chamber. The controller is configured not to perform an action below a predefined control error value, with a control error value defined for the pump and a different control error value defined for the valve.

Preferably, the harness includes a chest and shoulder strap.

Preferably, the harness also includes an arm strap.

Preferably, the above-mentioned control and pumping unit is programmed to maintain the preset constant pressure level in the cuff chamber, selected from among the predefined pressure settings.

Preferably, the control and pumping unit has a display, preferably an LCD display.

Preferably, the display is capable of showing the value of the current pressure in the cuff chamber or the actual time.

Preferably, the control and pumping unit is battery-powered.

Preferably, the control and pumping unit has an additional radio communication interface to communicate with other electronic devices, preferably an NFC (Near Field Communication) interface.

According to the invention, the method of controlling pressure in the pneumatic compression bandage including a cuff with a chamber to be filled with pressurized fluid, the harness to fasten the cuff in the wound area and a control and pumping unit connected pneumatically to the chamber in the cuff via a connection hose. The control and pumping unit contains an electrical pump, an electromechanical valve connected electrically with the control and pumping unit, a pressure sensor connected electrically with the control and pumping unit, as well as the memory and a system able to process digital information, with the user interface in the form of buttons, light and sound alarm devices. The method according to the invention is characterized by the fact that the pressure in the cuff chamber is controlled by a control algorithm of PID type, comprising a proportional, integral and derivative response, by means of a controller, consisting in controlling the electrical pump to increase pressure in the cuff chamber, while the valve is controlled to reduce pressure in the said chamber. The controller is configured not to perform an action below a predefined control error value, with a control error value defined for the pump and a different control error value defined for the valve.

The pressure control method in the pneumatic compression bandage covers also all the below stages:
A) placing the pneumatic compression bandage cuff centrally over the medical device implanted in the patient's body to ensure it covers the whole implanted medical device and the implantation site as much as possible;
B) fastening the cuff to the patient's body using the harness;
C) tightening the harness until the patient feels slight pressure at the site of the implanted medical bandage;
D) starting the pneumatic compression bandage;
E) checking the correct operation of the pneumatic compression bandage.

Stages E covers the following activities:
- inflating the cuff chamber to the first non-zero pressure setting in over a dozen of seconds;
- deflating the cuff chamber to the pressure near 0 mmHg in a couple of seconds;
- reporting the device emergency state if the PID controller, controlling the pump and the valve, fills the cuff chamber to the non-zero pressure setting within the time longer than the preset maximum adjustment time, preferably it is 60 s, or the pressure value in the cuff chamber fluctuates in the time longer than the preset maximum adjustment time, preferably it is 60 s.

The device emergency state is reported by a sound or light signal.

The memory of the control and pumping unit stores the pressure values in the cuff chamber at the intervals lower than or equal to 60 minutes, even more preferably - every 30 minutes, and even the most preferably - every 15 minutes.

Using the PID controller in the solution of the pneumatic compression bandage implies specific benefits for the device functionality and usability, including:
- rarer and shorter activation of the pump and the valve -> smaller battery use, greater patient's comfort during rest periods (e.g. when sleeping),
- more effective and more accurate reaching and maintenance of the preset pressure value and, consequently, compression force.

The PID controller used is one of 4 known controller types (P,PI,PD,PID). The PID controller, owing to the fact of using the three modules simultaneously (P for proportional, I for integral and D for derivative) is characterized by the shortest adjustment time in the set of controllers (the time from changing the selected pressure setting or from the interference occurrence to maintaining the preset level value) and the lowest control deviation (the difference between the preset level value and the measured one). In the pneumatic compression bandage, there is a divided actuating system used according to the invention. The controller controls the pump to increase pressure by switching it on, when the pressure value higher than or equal to the preset one is reached, the pump is switched off, and if the value is higher, the electrovalve is opened. Such a control system may be vulnerable to hazardous oscillations likely to result in fast-changing electrical signals and incorrect operation of the device (unstable pressure in the cuff chamber). Selecting the control parameters of the PID controller for systems with unforeseeable characteristics, e.g. a human body, is a difficult task. During the operation, the pneumatic compression bandage will be exposed to the disturbances of the pressure value in the cuff chamber resulting from the ordinary movements of the individual wearing it. To ensure the correct operation and the shortest time to achieve the pressure value in the cuff chamber preset by a doctor, the PID controller is used with control parameters selected experimentally. Thus, the shortest time to achieve the required pressure value in the chamber is obtained. When a patient moves and changes their body position and, consequently, the pressure in the chamber, the control and pumping unit will bring the pressure in the device chamber to the preset pressure value using the PID controller in the shortest time possible. Thus, the time when the wound compression is different than recommended is the shortest.

### The preferable embodiments of the invention

Now, the invention will be closer presented in the preferable embodiments, with reference to the attached drawings, in which:
Fig. 1 presents the control system diagram.

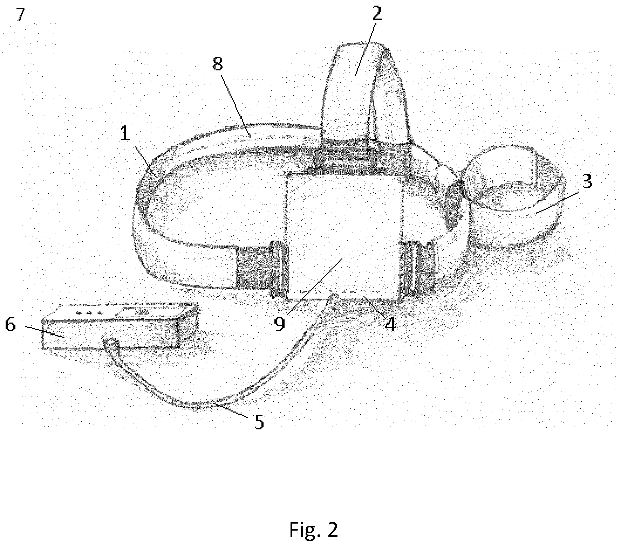

Fig. 2 presents the front view of the pneumatic compression bandage in the first preferable embodiment.

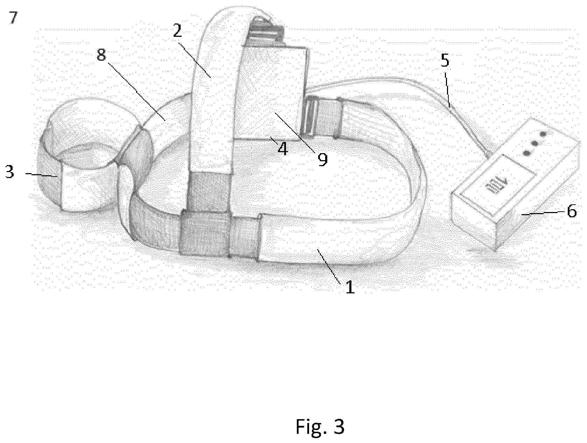

Fig. 3 presents the rear view of the pneumatic compression bandage in the first preferable embodiment.

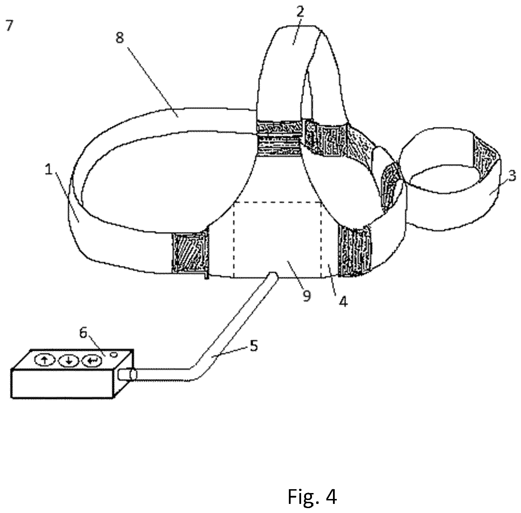

Fig. 4 presents the front view of the pneumatic compression bandage in the second preferable embodiment.

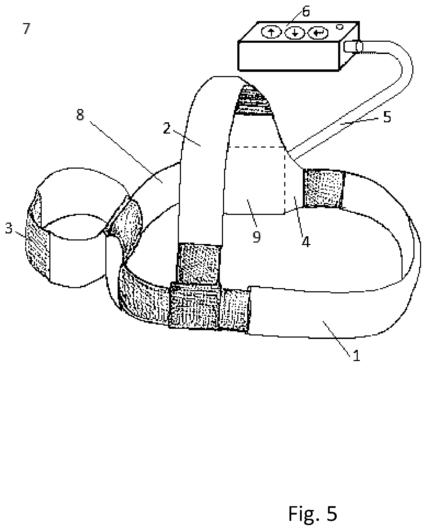

Fig. 5 presents the rear view of the pneumatic compression bandage in the second preferable embodiment.

The pneumatic compression bandage is a medical device intended for patients after the implantation procedure of medical devices used in cardiac electrotherapy to reduce the hemorrhagic complication risk in the implantation site. The pneumatic compression bandage is particularly recommended in patients using a double antiplatelet therapy or an oral anti-coagulation therapy (VKA, NOAC) in the periprocedural period.

The pneumatic compression bandage includes: a harness, a compression cuff with a connection hose, a pumping unit in a polymer housing (ABS) and two AA 1.5 V batteries delivered separately (included in the packaging).

**The harness 8** is composed of fastening straps 1, 2, 3 with a special structure which enable to adjust the compression cuff 4 correctly to the medical device implantation site. The harness 8 is made from a material ensuring comfortable and hygienic use, as well as protecting the skin from chafing and abrasion. The entire harness is disposable use.

**The compression cuff 4** with the connection hose 5 is the proper component of the pneumatic compression bandage 7, responsible for the compression of the site, i.e. the place where the medical device for cardiac electrotherapy was implanted. The cuff is composed of the chamber 9 of the cuff 4 and a trimming enabling to merge the cuff with the harness 8. The compression cuff 4 with the connection hose 5 is disposable use.

**The pumping unit 6** with an ABS polymer housing and batteries. The pumping unit has a membrane keypad which enables to control and manage the device operation. Replaceable alkaline batteries AA 1.5V are supplied separately (included in the packaging). Once the operation is completed, batteries should be removed (and disposed of in line with the applicable regulations). The ABS housing ensures further protection of the pump unit against mechanical damage, impact and scratches. The pumping unit is disposable use.

The pneumatic compression bandage 7 is designed to restrict everyday activities of the patient to the smallest degree possible, preventing any hemorrhagic complications in the site where the medical device was implanted.

The compression bandage 7 ensures the constant compression force, selected by the user, in the compression cuff due to the operation of the pump and the valve. The control is automatic thanks to the control algorithm implemented in the software.

### Definitions

Power supply system - this is the part of the electronic system which supplies voltage to the individual electronic system components.
Microcontroller - this is a programmable system used in the device, containing the device programme and controlling its operation.
Chamber 9 of the cuff 4 or, in other words, cushion 9 of the cuff 4 - the part of the cuff 4 in the Form of an inflatable rubber chamber, which the pressure control system is connected to
Pressure control system - is a set of components composed of a pump, valve, pressure sensor and tubes connecting it with the cuff cushion
Pump-is a component of the pressure control system pumping air to the harness chamber
Valve - is a component of the pressure control system enabling to release excess air from the system and reduce pressure in the cuff cushion
Pressure sensor- is a component of the pressure control system enabling to measure the current pressure in the cuff cushion
The control and pumping unit 6 termed also the pumping unit 6 - a part of the device in the polymer housing, containing a microcontroller and the pressure control system
Pressure - it is a current pressure in the pressure control system
Pressure indicator - it is a field in the software which stores the current value of pressure in the pressure control system
LED light indication - LED operation with a specific frequency where the LED voltage is low for one half of the time and high for the other half, wherein the number of light signals specified as the number of LED operation periods
Pressure setting - this is one of predefined pressure level values in the cuff cushion corresponding to the compression force expected by the user
Pressure sensor calibration - a calibration process consisting in calculating the correct value of the pressure indicator in the pressure control system based on the value returned by the pressure sensor
Settings calibration to the compression force - a calibration process consisting in adapting the pressure in the cuff cushion to the appropriate levels of the compression force of the cuff on the patient's body
State - this is a set of device information which enables to specify the position in the device operation scenario
The device operation scenario - this is the expected sequence of the device state changes
Self-Check - a test carried out automatically by the device to check its correct operation and the correctness of the stored dates
Date and time of the first start- this means a field in the software where the date and time saved following the successful completion of the first-started Self-Check test are kept
Calibration date and time - this means a field in the software which stores a date and time saved after the calibration process is completed
Current date and time - this means a field in the software which stores the current date and time of the device
PID - this is a part of the software controlling the valve and pump based on the pressure indicator and pressure setting values. It is a controller.

There are 4 basic types of controllers used in practice:
P controller (proportional).
   - the simplest of P, PI, PD, PID controllers
   - the P controller usually operates slightly with oscillations and even when it approaches the preset value, it will always be characterized by a control deviation (a difference between the preset signal value and the output signal value).
   - increasing the value of Kp coefficient results in a more accurate operation of the control system, but also an increased tendency to unstable (oscillatory) operation.
PI controller (proportional and integral one).
   - adding the I module to the P one eliminates slow-changing interferences and the determined error in exchange for the longer adjustment time than in the P controller
   - increased Kp gain and shortened integration time Ti results in a faster controller operation with simultaneous proximity to the stability limit (inclination to oscillate).
PD controller (proportional and derivative one).
   - adding the D module to the P one accelerates the controller operation but increases its vulnerability to the measurement noise;
   - PD controllers give a non-zero error which grows with the increase in the controller Kp gain;
   - operation of the derivative module prevents rapid error changes which stabilizes the control system operation.
PID controller:
   It is a hybrid of all the above components.

### Preliminary operating conditions

The device should be supplied from the main battery supply system and have an extra source of battery power supply, enabling to provide power supply to the integrated device clock to maintain the current date and time in the device.

The PID controller programmed in the device, controlling the pump and valve system to control pressure, should be responsible for controlling the compression force exerted by the cuff cushion.

Pressure settings are predefined.

The compression force can be selected manually by controlling the pressure.

### How to use

The device is switched on by pressing and holding the OK button for a prolonged time, e.g. 3 s. The device should be able to be started normally (to ensure normal operation) only after both calibration processes are completed. Once switched on, the device should enter the state when the Self-Check is carried out automatically.

### Self-Check test

It consists in undergoing the automatic test of the device correct operation. This test should be carried out by the device immediately after it is started. Depending on the start parameter value, the appropriate option of the Self-Check is selected.

During the first start, the device carries out the Self-Check procedure where the following parameters are checked in a sequence:
- battery status
- pressure sensor calibration status
- settings calibration to the compression force status
- current calibration status
- correct pump operation - inflating the cuff cushion to the first non-zero pressure setting in several seconds
- correct valve operation - discharging fluid from the cuff cushion (after an earlier pump test) to the pressure near 0 mmHg in a couple of seconds)
- correct operation of the PID controller - the control time exceeded - if the controller is not able to make the device reach the first non-zero setting and stabilize the pressure during the preset maximum control time, preferably 60 seconds, the device will report an emergency state.

### Device normal operation

Following the Self-Check tests, the device awaits the operation start. After the OK button is pressed, the device selects the first value in the setting sequence as the pressure setting and enables the user to select any of the predefined settings. The device checks if the pressure control error falls is within the limits of the pump and valve control errors. If it is not the case, the device starts the operation of the PID controller to reach the required pressure setting, avoiding pressure oscillations in the cuff cushion. The pressure is controlled by the operation of the pump and the valve. Summarizing, during the normal operation, the device checks the pressure in the cuff cushion and starts to control when the pressure is too high or too low. The PID controller itself operates based on a specific control strategy.

The control strategy is the adopted way of controlling and reaching the preset value in a way limiting the use of the valve because of its high inertia.

The device should have a permissible control error implemented, being the control error value below which the controller should not perform an action, with different error values defined for the pump (e.g. 300 Pa) and different for the valve (e.g. 500 Pa).

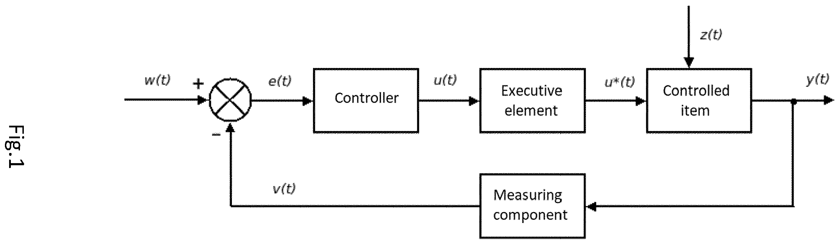

Fig. 1 depicts an illustrative diagram of the control system where:
- Controller - the PID controller implemented in the microcontroller
- Executive element - the pump and the valve
- The controlled item - the chamber 9 of the cuff 4
- Measuring component - the pressure sensor
- w(t) - the controller setting (e.g. 3,000 Pa)
- e(t) - the control error (the difference between the setting and the measured pressure)
- u(t) - output signal of the controller
- y(t) - the pressure in the cuff cushion
- v(t) - the pressure measured by the pressure sensor
- u*(t) - output signal of the executive element
- z(t) - external factors: forces affecting the controlled item (e.g. pressing by hand)

### PID control strategy

Control with energy quanta. The control consists in selecting quanta of electricity from the power supply delivered to the valve and pump. The sampling frequency is constant.

Pressure error e(t) between the pressure sample v(t) and the setting w(t) is analyzed by the PID controller. If the error value belongs to the permissible control error range, we stop the control. Depending on whether the error is positive or negative, we decide to control the pump or the valve. The controller returns the u(t) value in the Form of the valve or pump launching time. Thus, the speed of reaching the steadiness is achieved basing on the Kp, Ki and Kd parameters of the PID controller. Because of such control, we get quanta of energy sent in the Form of pressure to the cuff if the y(t) value deviates from w(t) a lot.

### Device use procedure

The cuff 4 with the harness 8 of the pneumatic compression bandage 7 is first applied in the operating theater by the doctor or another person authorized to carry out such activities immediately after the medical device (e.g. a stimulator or a cardioverter) implantation procedure is completed and after the wound is protected with a standard, sterile dressing. The procedure of applying the pneumatic compression bandage:
1) Ensure the postoperative wound is protected correctly with a sterile dressing
2) Place the compression cuff 4 of the pneumatic compression bandage 7 centrally over the implanted medical device to ensure it covers the entire implanted device and the implantation site as much as possible.
3) In the above position, the compression cuff 4 should be fixed using the adjustment of straps 1, 2, 3 of the harness 8. First, adjust the length of the chest strap 1, then of the shoulder strap 2 to ensure stable and fixed location of the cuff over the implantation site. Finally, fix the arm strap 3 preventing arm abduction.
4) The harness 8 should be fitted tight, i.e. the patient should feel a slight pressure on the area of the stimulator/cardioverter site even before the chamber 9 of the compression cuff 4 is filled automatically.

To start the pumping unit of the pneumatic compression bandage, press the OK button of the inactive device and hold it for 3 seconds. If the device is started for the first time, it undergoes an extended automatic Self-Check test. In any other case the device carries out solely the fast automatic Self-Check test (lasting ca. 1 second). Once the test is completed, the device reports its readiness for the operation by a repeating sequence of the green LED lighting: 2 seconds lit and 2 seconds break. Then, press OK to start the device operation.

If the device is started again after its parameters have been set and the keypad has been locked, right after the Self-Check test it starts to operate with the preset parameters and the device keypad is locked automatically.

The device has 6 predefined settings of the compression force. The compression force can be increased/decreased by pressing the "UP"/"DOWN" button when the device operates with the unlocked keypad. The device indicates the currently selected setting by the number of the green LED blinks (0 to 5). To confirm the selection, press "OK". The device will automatically control the pressure in the cuff to the selected compression force.

To switch on the manual mode, press the appropriate combination of buttons when the device operates with an unlocked keypad, e.g. pressing and holding the "UP" and "DOWN" buttons simultaneously for 3 seconds. It is indicated by 3 rapid beeps. The compression force is controlled by pressing and holding the button: "UP" increases the compression force by adding more air to the compression cuff and "DOWN" decreases the compression force by opening the discharge valve. The compression force value set by the above control should be accepted by pressing the "OK" button which will result in turning on the automatic mode in which the pumping unit will maintain the constant compression force set with the above programming. Pressing the "UP" or "DOWN" button will result in returning to the last predefined value in the automatic mode and a shift to the adjacent setting (higher / lower depending on the button pressed).

The pneumatic compression bandage provides the possibility of locking the keypad. The device with a locked keypad maintains the appropriate pressure in the cuff but no longer responds to other key combinations (apart from the unlocking combination). This prevents any changes of settings by unauthorized people. The keypad can be locked during normal device operation by pressing an appropriate combination of buttons, e.g. pressing and holding the "UP" button and pressing the "DOWN" button 3 times simultaneously (the keypad is unlocked in the same way).

The pumping unit is switched off by pressing and holding the "OK" button for a longer time, e.g. 3 seconds. If the device keypad is locked, the device can be switched off by removing the cuff connection hose from the control unit, waiting ca. 1 minute until the device starts emitting the emergency sound signal and the red LED is lit, then pressing the "OK" button and holding it for 3 seconds. The device will switch off.

### Starting and setting the parameters of the pneumatic compression bandage on a patient.

The procedure describes the start and selection of the compression force of the device immediately after the operation.
1) Connect the cuff connection hose to the pumping unit.
2) Switch the device on.
3) Wait ca. 1 minute until the device automatically completes the Self-Check test (the successful Self-Check is signaled by the green LED lighting in the sequence of 2 seconds lit and 2 seconds break).
4) Switch the device off.
5) Put on the compression cuff
6) Switch the device on again.
7) Press the "OK" button.
8) Select the appropriate compression force in the automatic or manual mode
9) Wait for a while until the device stabilizes pressure in the cuff and ensure the site compression is appropriate.
10) Lock the keypad.
11) After the above activities are completed, the device is ready for further automated operation and it can be handed over to the patient.

### Procedure for the replacement of sterile dressing

When the pneumatic compression bandage 7 is used, it may be necessary to replace the standard sterile dressing. Before doing it, switch off the pumping unit. Next, remove the compression cuff 4 and change the sterile dressing. After you put on a new sterile dressing, put the cuff 4 on again (as described above), connect the cuff connection hose 5 to the pumping unit 6 and start the device. The started pneumatic compression bandage 7 seeks to reach the setting before it was switched off and maintains it.

### Preferred power supply methods

In the pneumatic compression bandage solely the battery/rechargeable battery power supply is permitted. Preferably, power is supplied to the device from 2 AA 1.5 V alkaline batteries. If the device batteries are discharged, they must be replaced by new ones. Low battery is indicated by a relevant emergency signal. To replace batteries, switch off the device, remove the cover at the device bottom, remove the empty batteries and replace them with new ones. Then close the cover, connect the cuff to the pumping unit again and start the device. If the battery level is too low, the device will not start normal operation. After the operation is completed, remove batteries from the device.

### Communications with external devices

The control and pumping unit 6 has an integrated wireless communication interface using radio waves, preferably in the NFC (Near Field Communication) standard. Using the device with the NFC communicator and a controlling application, e.g. a smartphone, the user can communicate with the control and pumping unit 6 to send, read or modify the current configuration of the unit 6 settings. Preferably, this communication will enable also to send or read the information on the patient and therapy administered. Communication using the NFC standard is a touchless, hygienic communication method which enables to read the data saved in the control and pumping unit without the need to touch the device, open the housing or connect the cables.

### Possible emergency situations and remedies

During the device operation an emergency situation can occur which is indicated by a red LED lighting and an emergency beep. In such a case the device switches off the pump automatically, opens the valve and switches to the emergency state indication mode. In that mode, the device operation is limited to the periodic repetition of the emergency signal. The emergency state mode can be quit by switching the device off. Possible emergency states:

| **Emergency signal** | **Failure** | **Remedies** |
|---|---|---|
| - periodic blinking of the red LED: a characteristic sequence of blinking, e.g. 0.5 seconds lit and 0.5 seconds break. | - battery discharged | - replace batteries and restart the device |
| A beep signal is emitted simultaneously with blinking | | |
| - periodic blinking of the red LED: a characteristic sequence of blinking (different than the previous one) e.g. 1 second lit and 1 second break; | - disconnected cuff | - check the cuff connection hose connection to the device and restart the device, if the emergency state persists, call the healthcare practitioners or contact technical support |
| A beep signal is emitted simultaneously with the LED lit | | |
| - other emergency signals (different than the above sequences) - the sequence of the red LED blinking with 2 seconds interval; | - other failures | - call the healthcare practitioners or contact technical support |
| A beep signal is emitted simultaneously with the blinking | | |

## Claims

1. A pneumatic compression bandage (7) intended particularly for preventing hemorrhagic complications following medical device implantations in cardiac electrotherapy, including a cuff (4) with a chamber (9) to be filled with a pressurized fluid, a harness (8) used to fasten the cuff (4) in a wound area and a control and pumping unit (6) connected pneumatically to the chamber (9) in the cuff with a connection hose (5), whereby this control and pumping unit (6) includes an electrical pump, an electromechanical valve connected electrically with the control and pumping unit, a pressure sensor connected electrically with the control and pumping unit (6), as well as memory and a system processing digital information, and a user interface in the form of buttons, light and sound indicators, **characterized in that** the pressure controller in the cuff (4) chamber (9) is a PID controller, meaning it includes a proportional (P), integral (I) and derivative (D) modules, to control the electric pump to increase pressure in the cuff (4) chamber (9) and to control the valve to reduce pressure in the cuff (9) chamber (4), wherein the controller is configured not to perform an action below a predefined control error value, with a control error value defined for the pump and a different control error value defined for the valve.

2. The bandage according to claim 1, **characterized in that** the fact that the harness (8) has a chest strap (1) and a shoulder strap (2).

3. The bandage according to claim 2, **characterized in that** the fact that the harness includes also an arm strap (3).

4. The bandage according to claim 1, 2 or 3, **characterized in that** the fact that the control and pumping unit (6) is programmed to maintain the preset constant pressure level in the cuff (4) chamber (9), selected from among the predefined settings.

5. The bandage according to any one of the preceding claims, **characterized in that** the fact that the control and pumping unit (6) has a display, preferably an LCD display.

6. The bandage according to claim 5, **characterized in that** the fact that the display is capable of showing the value of the current pressure in the cuff (4) chamber (9) or the actual time.

7. The bandage according to any one of the preceding claims, **characterized in that** the fact that the control and pumping unit (6) is battery-powered.

8. The bandage according to any one of the preceding claims, **characterized in that** the fact that it has also a radio communication interface to communicate with other electronic devices, the NFC (Near Field Communication) interface which is an advantage.

9. A method of pressure control in a pneumatic compression bandage (7), comprising a cuff (4) with a chamber (9) to be filled with a pressurized fluid, a harness (8) used to fasten the cuff (4) in a wound area and a control and pumping unit (6) connected pneumatically to the chamber (9) in the cuff (4) via a connection hose (5), whereby the said control and pumping unit (6) includes an electrical pump, an electromechanical valve connected electrically with the control and pumping unit, a pressure sensor connected electrically with the control and pumping unit, as well as memory and a system processing digital information, with the user interface in the form of buttons and light and sound indicators, **characterized in that** the fact that the pressure in the cuff (4) chamber (9) is controlled by the PID control algorithm, comprising a proportional (P), integral (I) and derivative (D) response, by means of a controller, consisting in controlling the electrical pump to increase pressure in the cuff (4) chamber (9), while the valve is controlled to reduce pressure in the said cuff (4) chamber (9), wherein the controller is configured not to perform an action below a predefined control error value, with a control error value defined for the pump and a different control error value defined for the valve.

10. The method according to claim 9, **characterized in that** the fact that it covers all of the below-mentioned stages:
(A) placing the pneumatic bandage (7) cuff (4) centrally over the medical device implanted in the patient's body to ensure it covers the whole implanted medical device and the implantation site as much as possible;
B) fastening the cuff (4) to the patient's body using the harness (8);
C) tightening the harness (8) until the patient feels slight pressure at the site of the implanted medical device;
D) starting the pneumatic compression bandage (7);
E) checking the correct operation of the pneumatic compression bandage (7).

11. The method according to claim 10, **characterized in that** the fact that step E comprises the following activities:
- inflating the cuff (4) chamber (9) to the first non-zero pressure setting in over a dozen of seconds;
- discharging fluid from the cuff (4) chamber (9) to the pressure near 0 mmHg in a couple of seconds;
- reporting the device emergency state if the PID controller controlling the pump and the valve fills the cuff (4) chamber (9) to the non-zero pressure setting within the time longer than the preset
maximum adjustment time, preferably it is 60 s, or the pressure value in the cuff (4) chamber (9) fluctuates in the time longer than the preset maximum adjustment time, preferably it is 60 s.

12. The method according to claim 11, **characterized in that** the fact that a device emergency state is indicated by a sound or light indication.

13. The method according to any one of the claims from 9 to 12, **characterized in that** the fact that the memory stores the pressure values in the cuff (4) chamber (9) at the intervals lower than or equal to 60 minutes, preferably every 30 minutes and even more preferably every 15 minutes.

## Patentansprüche

1. Pneumatischer Kompressionsverband (7), insbesondere zur Vorbeugung von hämorrhagischen Komplikationen nach Implantation medizinischer Geräte in der kardialen Elektrotherapie, bestehend aus einer Manschette (4) mit einer Kammer (9), die mit einer Druckflüssigkeit zu füllen ist, ein Geschirr (8), das zur Befestigung der Manschette (4) in einem Wundbereich dient, und eine Steuer- und Pumpeinheit (6), die über einen Verbindungsschlauch (5) pneumatisch mit der Kammer (9) in der Manschette verbunden ist, wobei diese Steuer- und Pumpeinheit (6) eine elektrische Pumpe, ein elektrisch mit der Steuer- und Pumpeinheit verbundenes elektromechanisches Ventil, einen elektrisch mit der Steuer- und Pumpeinheit (6) verbundenen Drucksensor sowie einen Speicher und ein System zur Verarbeitung digitaler Informationen und eine Benutzerschnittstelle in Form von Tasten, Licht- und Tonanzeigen, **dadurch gekennzeichnet, dass** der Druckregler in der Kammer (9) der Manschette (4) ein PID-Regler ist, d.h. er umfasst ein ein proportionales (P), integrales (I) und abgeleitetes (D) Modul, um die elektrische Pumpe zu steuern, um den Druck in der Kammer (9) der Manschette (4) zu erhöhen, und um das Ventil zu steuern, um den Druck in der Kammer (4) der Manschette (9) zu verringern, wobei der Regler so konfiguriert ist, dass er unterhalb eines vordefinierten Steuerfehlerwertes keine Aktion ausführt, wobei ein Steuerfehlerwert für die Pumpe und ein anderer Steuerfehlerwert für das Ventil definiert ist.

2. Bandage nach Anspruch 1, **dadurch gekennzeichnet, dass** das Geschirr (8) einen Brustgurt (1) und einen Schultergurt (2) aufweist.

3. Bandage nach Anspruch 2, **dadurch gekennzeichnet, dass** das Geschirr auch einen Armgurt (3) aufweist.

4. Bandage nach Anspruch 1, 2 oder 3, **dadurch gekennzeichnet, dass** die Steuer- und Pumpeinheit (6) so programmiert ist, dass sie das voreingestellte konstante Druckniveau in der Kammer (9) der Manschette (4) aufrechterhält, das aus den vordefinierten Einstellungen ausgewählt wird.

5. Bandage nach einem der vorhergehenden Ansprüche, **dadurch gekennzeichnet, dass** die Steuer- und Pumpeinheit (6) ein Display, vorzugsweise ein LCD-Display, aufweist.

6. Bandage nach Anspruch 5, **dadurch gekennzeichnet, dass** das Display in der Lage ist, den Wert des aktuellen Drucks in der Kammer (9) der Manschette (4) oder die aktuelle Zeit anzuzeigen.

7. Bandage nach einem der vorhergehenden Ansprüche, **dadurch gekennzeichnet, dass** die Steuer- und Pumpeinheit (6) batteriebetrieben ist.

8. Die Bandage nach einem der vorhergehenden Ansprüche, **dadurch gekennzeichnet, dass** sie auch eine Funkkommunikationsschnittstelle zur Kommunikation mit anderen elektronischen Geräten aufweist, wobei die NFC-Schnittstelle (Near Field Communication) von Vorteil ist.

9. Verfahren zur Druckregelung in einem pneumatischen Kompressionsverband (7), bestehend aus einer Manschette (4) mit einer mit einer Druckflüssigkeit zu füllenden Kammer (9), einem Geschirr (8) zur Befestigung der Manschette (4) in einem Wundbereich und einer Steuer- und Pumpeinheit (6), die über einen Verbindungsschlauch (5) pneumatisch mit der Kammer (9) in der Manschette (4) verbunden ist, wobei die genannte Steuer- und Pumpeinheit (6) eine elektrische Pumpe, ein mit der Steuer- und Pumpeinheit elektrisch verbundenes elektromechanisches Ventil, einen mit der Steuer- und Pumpeinheit elektrisch verbundenen Drucksensor sowie einen Speicher und ein System zur Verarbeitung digitaler Informationen umfasst, mit der Benutzerschnittstelle in Form von Tasten und Licht- und Tonanzeigen, **dadurch gekennzeichnet, dass** der Druck in der Kammer (9) der Manschette (4) durch den PID-Regelalgorithmus, der eine proportionales (P), integrales (I) und abgeleitetes (D) Verhalten umfasst, mittels eines Reglers gesteuert wird, der darin besteht, die elektrische Pumpe zu steuern, um den Druck in der Kammer (9) der Manschette (4) zu erhöhen, während das Ventil gesteuert wird, um den Druck in der Kammer (9) der Manschette (4) zu verringern, wobei der Regler so konfiguriert ist, dass er keine Aktion unterhalb eines vordefinierten Steuerfehlerwertes ausführt, wobei ein Steuerfehlerwert für die Pumpe und ein anderer Steuerfehlerwert für das Ventil definiert ist.

10. Verfahren nach Anspruch 9, **dadurch gekennzeichnet, dass** es die nachstehenden Schritte umfasst:
(A) Anlegen der Manschette (4) der pneumatischen Bandage (7) mittig über das in den Körper des Patienten implantierte medizinische Gerät, um sicherzustellen, dass sie das gesamte implantierte medizinische Gerät und die Implantationsstelle so weit wie möglich abdeckt;
B) Befestigung der Manschette (4) am Körper des Patienten mit Hilfe des Geschirrs (8);
C) Anziehen des Geschirrs (8), bis der Patient einen leichten Druck an der Stelle des implantierten medizinischen Geräts spürt;
D) Anlegen des pneumatischen Kompressionsverbands (7);
E) Überprüfen der korrekten Funktion des pneumatischen Kompressionsverbands (7).

11. Verfahren nach Anspruch 10, **dadurch gekennzeichnet, dass** der Schritt E die folgenden Tätigkeiten umfasst:
- Aufblasen der Kammer (9) der Manschette (4) auf den ersten Druckwert ungleich Null in mehr als einem Dutzend Sekunden;
- Ablassen von Flüssigkeit aus der Kammer (9) der Manschette (4) auf den Druck nahe 0 mmHg in einigen Sekunden;
- Melden des Notzustands der Vorrichtung, wenn der PID-Regler, der die Pumpe und das Ventil steuert, die Kammer (9) der Manschette (4) bis zu dem von Null verschiedenen Druck füllt.
maximale Einstellzeit, vorzugsweise 60 s, oder der Druckwert in der Kammer (9) der Manschette (4) schwankt in der Zeit länger als die voreingestellte maximale Einstellzeit, vorzugsweise 60 s, beträgt.

12. Verfahren nach Anspruch 11, **dadurch gekennzeichnet, dass** die Tatsache, dass ein Gerätenotzustand vorliegt, durch eine Ton- oder Lichtanzeige angezeigt wird.

13. Verfahren nach einem der Ansprüche 9 bis 12, **dadurch gekennzeichnet, dass** der Speicher die Druckwerte in der Kammer (9) der Manschette (4) in Intervallen speichert, die kleiner oder gleich 60 Minuten sind, vorzugsweise alle 30 Minuten und noch bevorzugter alle 15 Minuten.

## Revendications

1. Un bandage compressif pneumatique (7) destiné notamment à prévenir les complications hémorragiques suite à l'implantation d'un dispositif médical en électrothérapie cardiaque, comprenant une manchette (4) avec une chambre (9) à remplir d'un fluide sous pression, un harnais (8) utilisé pour fixer la manchette (4) dans une zone de plaie et une unité de régulation et de pompage (6) reliée pneumatiquement à la chambre (9) dans la manchette avec un tuyau de raccordement (5), dans lequel cette unité de régulation et de pompage (6) comprend une pompe électrique, une vanne électromécanique reliée électriquement à l'unité de régulation et de pompage, un capteur de pression relié électriquement à l'unité de régulation et de pompage (6), ainsi qu'une mémoire et un système de traitement des informations numériques, et une interface utilisateur sous la forme de boutons, d'indicateurs lumineux et sonores,**caractérisé en ce que** le régulateur de pression dans la chambre (9) de la manchette (4) est un régulateur PID, c'est-à-dire qu'il comprend des modules proportionnel (P), intégral (I) et dérivé (D), pour réguler la pompe électrique afin d'augmenter la pression dans la chambre (9) de la manchette (4) et pour réguler la vanne afin de réduire la pression dans la chambre (4) de la manchette (9), dans lequel le régulateur est configuré pour ne pas effectuer une action en dessous d'une valeur d'erreur de régulation prédéfinie, une valeur d'erreur de régulation étant définie pour la pompe et une valeur d'erreur de régulation différente étant définie pour la vanne.

2. Le bandage selon la revendication 1, **caractérisé en ce que** le harnais (8) a une sangle de poitrine (1) et une sangle d'épaule (2).

3. Le bandage selon la revendication 2, **caractérisé en ce que** le harnais comprend également une sangle de bras (3).

4. Le bandage selon la revendication 1, 2 ou 3, **caractérisé en ce que** l'unité de régulation et de pompage (6) est programmée pour maintenir le niveau de pression constant prédéfini dans la chambre (9) de la manchette (4), sélectionné parmi les réglages prédéfinis.

5. Le bandage selon l'une quelconque des revendications précédentes, **caractérisé en ce que** l'unité de régulation et de pompage (6) a un écran, de préférence un écran LCD.

6. Le bandage selon la revendication 5, **caractérisé en ce que** l'écran est capable d'afficher la valeur de la pression actuelle dans la chambre (9) de la manchette (4) ou l'heure actuelle.

7. Le bandage selon l'une quelconque des revendications précédentes, **caractérisé en ce que** l'unité de régulation et de pompage (6) est alimentée par batterie.

8. Le bandage selon l'une quelconque des revendications précédentes, **caractérisé en ce que** le fait qu'il dispose également d'une interface de communication radio pour communiquer avec d'autres dispositifs électroniques, l'interface NFC (Near Field Communication), est un avantage.

9. Un procédé de régulation de pression dans un bandage compressif pneumatique (7), comprenant une manchette (4) avec une chambre (9) à remplir d'un fluide sous pression, un harnais (8) utilisé pour fixer la manchette (4) dans une zone de plaie et une unité de régulation et de pompage (6) reliée pneumatiquement à la chambre (9) dans la manchette (4) par l'intermédiaire d'un tuyau de raccordement (5), dans lequel ladite unité de régulation et de pompage (6) comprend une pompe électrique, une vanne électromécanique reliée électriquement à l'unité de régulation et de pompage, un capteur de pression relié électriquement à l'unité de régulation et de pompage, ainsi qu'une mémoire et un système de traitement des informations numériques, avec l'interface utilisateur sous la forme de boutons et d'indicateurs lumineux et sonores,**caractérisé en ce que** la pression dans la chambre (9) de la manchette (4) est régulée par l'algorithme de régulation PID, comprenant une réponse proportionnelle (P), intégrale (I) et dérivée (D), au moyen d'un régulateur, consistant à réguler la pompe électrique pour augmenter la pression dans la chambre (9) de la manchette (4), tandis que la vanne est régulée pour réduire la pression dans ladite chambre (9) de la manchette (4), dans lequel le régulateur est configuré pour ne pas effectuer une action en dessous d'une valeur d'erreur de régulation prédéfinie, une valeur d'erreur de régulation étant définie pour la pompe et une valeur d'erreur de régulation différente étant définie pour la vanne.

10. Le procédé selon la revendication 9, **caractérisé en ce qu'**il couvre les étapes mentionnées ci-dessous :
A) placement de la manchette (4) du bandage pneumatique (7) au centre du dispositif médical implanté dans le corps du patient pour s'assurer qu'elle couvre autant que possible l'ensemble du dispositif médical implanté et le site d'implantation ;
B) fixation de la manchette (4) au corps du patient à l'aide du harnais (8) ;
C) serrage du harnais (8) jusqu'à ce que le patient ressente une légère pression au niveau du site du dispositif médical implanté ;
D) démarrage du bandage compressif pneumatique (7) ;
E) vérification du bon fonctionnement du bandage compressif pneumatique (7).

11. Le procédé selon la revendication 10, **caractérisé en ce que** l'étape E comprend les activités suivantes :
- le gonflage de la chambre (9) de la manchette (4) au premier réglage de pression non-nulle en plus d'une douzaine de secondes ;
- évacuation du liquide de la chambre (9) de la manchette (4) à la pression proche de 0 mmHg en quelques secondes ;
- signalement de l'état d'urgence du dispositif si le régulateur PID régulant la pompe et la vanne remplit la chambre (9) de la manchette (4) à la pression non-nulle dans un temps supérieur au temps de réglage maximal prédéfini,
de préférence égal à 60 s, ou si la valeur de pression dans la chambre (9) de la manchette (4) fluctue dans un temps supérieur au temps de réglage maximal prédéfini, de préférence égal à 60 s.

12. Le procédé selon la revendication 11, **caractérisé en ce que** l'état d'urgence du dispositif est indiqué par une indication sonore ou lumineuse.

13. Le procédé selon l'une quelconque des revendications de 9 à 12, **caractérisé en ce que** la mémoire stocke les valeurs de pression dans la chambre (9) de la manchette (4) à des intervalles inférieurs ou égaux à 60 minutes, de préférence toutes les 30 minutes et encore plus préférentiellement toutes les 15 minutes.
